# EUROPÄISCHE PATENTSCHRIFT

(11) **EP 2 135 624 B1**
(45) Veröffentlichungstag und Bekanntmachung des Hinweises auf die Patenterteilung: **08.08.2012**
(21) Anmeldenummer: 09007822.1
(22) Anmeldetag: 15.06.2009
(51) Int. Cl.: A61L 2/00, A61L 2/08, A61L 11/00, B01J 19/08, C02F 1/30

(54) **Verfahren und Vorrichtung zum Inaktivieren einer mikrobiologisch kontaminierten und Feststoffpartikel enthaltenden Masse mittels beschleunigter Elektronen**
Method and device to inactivate a mass microbiologically contaminated and containing solid particles using accelerated electrons
Procédé et dispositif d'inactivation d'une masse microbiologique contaminée et comprenant des particules de matière solide à l'aide d'électrons accélérés

(30) Priorität: 16.06.2008 DE 102008028545
(43) Veröffentlichungstag der Anmeldung: 23.12.2009
(73) Patentinhaber: Fraunhofer-Gesellschaft zur Förderung der angewandten Forschung e.V., 80686 München (DE); Glatt Systemtechnik GmbH, 01277 Dresden (DE); Glatt Ingenieurtechnik GmbH, 99427 Weimar (DE)
(72) Erfinder: Röder, Olaf, Dr., 01326 Dresden (DE); Flaske, Henrik, 01157 Dresden (DE); Wetzel, Christiane, Dr., 01212 Dresden (DE); Rögner, Frank-Holm, 01277 Dresden (DE); Kretzschmar, Ralf, 01309 Dresden (DE); Wegner, Thomas, 01109 Dresden (DE); Böber, Reinhard, 99425 Weimar (DE); Prahl, Wolfgang, 99094 Erfurt (DE)

(56) Entgegenhaltungen:
- WO-A-00/16615
- US-A- 3 988 588
- US-A- 4 230 947
- US-A- 5 451 790
- US-A1- 2001 042 841
- US-A1- 2007 248 486

## Beschreibung

Die Erfindung betrifft ein Verfahren und eine Vorrichtung zum Inaktivieren einer mikrobiologisch kontaminierten und Feststoffpartikel enthaltenden Masse mit beschleunigten Elektronen.

Mikrobiologisch kontaminierte Produkte bzw. Abfälle fallen vorrangig in der pharmazeutischen und biotechnologischen Industrie, im Zuge von Tierversuchen, bei der Tierkörperbeseitigung, bei der Abfall- und Abwasseraufbereitung sowie bei der tierischen Produktion in Form von Gülle an, um nur einige Beispiele zu nennen.

Biologisch kontaminierte flüssige oder feste Abfälle sind verschiedenen Biosicherheitsklassen zugeordnet. Bisher werden derartige Abfälle überwiegend chemisch oder thermisch inaktiviert, das heißt die mikrobiologische Kontamination in Form von Viren, Bakterien, Mikropilzen sowie ggf. Prionen wird beseitigt.

Unter dem Begriff "Inaktivieren" soll nachfolgend verstanden werden, dass die gesundheitsschädigende Wirkung von mikrobiologisch kontaminierten Bestandteilen einer Masse eliminiert wird. Dies kann einerseits bedeuten, dass die mikrobiologisch kontaminierten Bestandteile einer Masse sterilisiert werden. Andererseits kann es aber auch schon hinreichend sein, wenn die anhaftenden Mirkoorganismen derart beeinflusst werden, dass diese nicht mehr vermehrungsfähig sind bzw. dass die Anzahl vermehrungsfähiger Mikroorganismen verringert wird, um deren gesundheitsschädigende Wirkung zu unterbinden.

Die Art mikrobiologisch kontaminierter Abfälle reicht von wässrigen Flüssigkeiten über Hühnereier aus der Impfstoffproduktion bis hin zu Tierkörpern wie zum Beispiel Versuchsmäusen. Aber auch die aus der Tierhaltung stammende Gülle ist häufig mit Bakterien wie z. B. Salmonellen kontaminiert, die vor dem Ausbringen auf Felder inaktiviert werden sollte.

Je nach Art des Abfalls und der Kontamination (Gefährlichkeit der Viren, Bakterien, Mikropilzen oder Prionen) sind bekannte Methoden zum Inaktivieren mit Risiken und teilweise erheblichen Nachteilen verbunden. Insbesondere erfordern zum Beispiel thermische Methoden einen erheblichen Energieaufwand, da Temperaturen größer 70 °C oder sogar über 130 °C und lange Expositionszeiten erforderlich sind.

Eine weitere Methode ist die Inaktivierung im Zuge der Verbrennung. In diesem Fall wird das Produkt jedoch vernichtet und steht keiner weiteren Verwendung zur Verfügung.

Chemische Methoden, wie zum Beispiel die Inaktivierung von Abfällen aus der Impfstoffproduktion (z. B. angebrütete Hühnereier), beruhen oftmals auf einer Behandlung mit Säuren oder Basen. Dies ist mit dem Risiko verbunden, dass die Inaktivierung größerer Volumina nicht jedes Volumenelement erfasst. Auch eine Inline-Überwachung des Prozesses sowie eine schlüssige Validierung sind nicht möglich. Die Gewährleistung der Biosicherheit kann daher nur durch zyklische Probenentnahme erfolgen. Darüber hinaus ist diese Methode mit erheblichen Wartezeiten verbunden, um die erforderliche Mindesteinwirkzeit der Säuren oder Basen zu gewährleisten.

Ein erhebliches Problem beim Inaktivieren mikrobiologischer Kontaminationen ist oft damit verbunden, dass die Produkte bzw. Abfälle Feststoffe wie Knochen, Knorpel, Eierschalen und ähnliches enthalten. Herkömmliche Methoden sind nur begrenzt in diesen Feststoffbestandteilen wirksam, was zusätzliche Risiken in der Anwendung, dem Nachweis und bei der Entsorgung mit sich bringt.

Es ist bekannt, dass mittels beschleunigter Elektronen Keime bzw. Mikroorganismen abgetötet werden können. In DE 199 42 142 A1 wird beispielsweise Saatgut bei mehrfachdurchlauf im freien Fall mit beschleunigten Elektronen beaufschlagt, um Keime am Saatgut abzutöten. DE 20 2006 015 636 U1 beschreibt eine Lehre, bei der rieselfähige pflanzliche Produkte in einer Kammer mittels eines Gasstromes verwirbelt und währenddessen mit beschleunigten Elektronen beaufschlagt werden. Beiden Vorschlägen ist der Nachteil immanent, dass diese nicht für die Keimreduktion an Massen geeignet sind, die Flüssigkeitsbestandteile aufweisen.

Aus dem Bereich der Wasseraufbereitung sind Vorschläge bekannt, bei denen Flüssigkeiten zur Keimabtötung mit beschleunigten Elektronen beaufschlagt werden (WO 02/02466 A1, EP 0 931 765 A2, EP 0 024 487 A1, US 3,988,588). Bekannt sind auch Lösungen, bei denen eine Flüssigkeit zu einem schichtförmigen Volumen geformt wird, welches dann mit beschleunigten Elektronen beaufschlagt wird. US 4,230,941 schlägt vor, eine Flüssigkeit auf ein Reservoir anzuheben, von wo es über eine Kante läuft und als Wasservorhang hinunterfällt. Aus US 5.451,790 ist eine Vorrichtung bekannt, bei der eine Flüssigkeit zunächst in einen Behälter gefüllt wird und von dort durch einen schmalen Spalt in die Tiefe fließt. Im schmalen Spalt zu einem schichtförmigen Volumen geformt, wird die Flüssigkeit mit beschleunigten Elektronen beaufschlagt. Derartige Vorrichtungen und Verfahren sind wiederum nicht zur Behandlung von Flüssigkeiten geeignet, die mikrobiologisch kontaminierte Feststoffpartikel aufweisen, weil bei vorschlägen keine Maßnahmen ersichtlich sind, die das Sedimentieren von Feststoffpartikeln auf dem Boden von Rohren oder Behältern verhindern, wodurch die dort sedimentierten Feststoffpartikel entweder überhaupt nicht, zumindest jedoch nicht allseitig mit beschleunigten Elektronen beaufschlagt werden und dadurch nicht die gesamte Masse aus flüssigen und festen Bestandteilen inaktiviert wird.

Bei einer aus US 2001/0042841 A1 bekannten Bestrahlungsvorrichtung wird ein Material durch eine spaltförmige Röhre transportiert und in dieser Röhre mit beschleunigten Elektronen beaufschlagt, Dabei wird ein Bereich der Röhrenwandung durch das Elektronenaustrittsfenster eines an die Röhrenwandung angeflanschten Elektronengenerators gebildet, so dass die aus dem Elektronenfenster austretenden Elektronen ummittelbar auf das zu bestrahlende Material auftreffen. Nachteilig wirkt sich hierbei aus, dass bei beschädigtem Elektronenaustrittsfenster das zu bestrahlende Material in das Innere des Elektronengenerators aufgrund des darin vorherrschenden Vakuums gezogen und somit verunreinigt und gegebenenfalls mit Schadstoffen kontaminiert wird. Auch ist bei dieser Vorrichtung das Problem des Sedimentierens von Feststoff partikeln nicht behoben.

### Aufgabenstellung

Der Erfindung liegt daher das technische Problem zugrunde, ein Verfahren und eine Vorrichtung zum Inaktivieren einer mikrobiologisch kontaminierten und Feststoffpartikel enthaltenden Masse zu schaffen, mittels denen die Nachteile aus dem Stand der Technik überwunden werden können. Insbesondere sollen Verfahren und Vorrichtung für das Inaktivieren von Feststoffpartikel enthaltenden Massen geeignet sein, die auch Flüssigkeitsbestandteile aufweisen können.

Die Lösung des technischen Problems ergibt sich durch die Gegenstände mit den Merkmalen der Patentansprüche 1 und 8. Weitere vorteilhafte Ausgestaltungen der Erfindung ergeben sich aus den abhängigen Patentansprüchen.

Bei erfindungsgemäßen Verfahren und Vorrichtungen wird zunächst eine mikrobiologisch kontaminierte und Feststoffpartikel enthaltende Masse mittels einer geeigneten Einrichtung mit einem Geliermittel vermischt. Dabei kann die Feststoffpartikel enthaltende Masse entweder nur aus Festbestandteilen oder zusätzlich auch aus Flüssigbestandteilen bestehen. Beim Vermischen ist der Feststoffpartikel enthaltenden Masse so viel Geliermittel hinzuzufügen, dass eine breiartige Masse entsteht, die einerseits noch fließfähig bzw. pumpfähig ist und andererseits eine solche Konsistenz aufweist, dass die Feststoffpartikel in der breiartigen Masse derart fixiert sind, dass ein Sedimentieren der Feststoffpartikel nicht möglich ist.

Vorteilhaft ist es, wenn das Vermischen der Feststoffpartikel enthaltenden Masse mit dem Gellermittel derart erfolgt, dass die Feststoffpartikel nach dem Vermischen mit dem Gellermittel in der breiartigen Masse dispergiert vorliegen, so dass eine homogenisierte breiartige Masse entsteht, d. h. die Feststoffpartikel liegen innerhalb der breiartigen Masse vereinzelt und gleichmäßig verteilt vor.

Bei einer Ausführungsform wird beim Vermischen der Feststoffpartikel enthaltenden Masse neben einem Geliermittel auch noch eine Flüssigkeit, wie beispielsweise Wasser, hinzugefügt. Dies kann zum Beispiel vorteilhaft sein, wenn die Feststoffpartikel enthaltende Masse nur aus Feststoffpartikeln besteht bzw. wenn die mit Geliermittel vermischte Masse noch keine fließfähige bzw. pumpfähige Konsistenz aufweist.

Nachdem die Feststoffpartikel enthaltende Masse mit einem Geliermittel vermischt ist, wird die daraus entstehende breiartige Masse durch formgebende Mittel transportiert. Das Transportieren kann beispielsweise mittels Pump-, Saug- oder Presseinrichtungen erfolgen. In einem Bereich der formgebenden Mittel wird die breiartige Masse zu einem schichtförmigen Volumen mit einer Schichtdicke von 1 mm bis 3 mm geformt und dieses schichtförmige Volumen zumindest von einer Seite her mit beschleunigten Elektronen, herrührend von mindestens einem Elektronenstrahlerzeuger (auch Elektronenbeschleuniger genannt), beaufschlagt. Der Bereich der formgebenden Mittel, in dem die breiartige Masse zu dem schichtförmigen Volumen mit 1 mm bis 3 mm Schichtdicke formbar ist, wird nachfolgend auch als Spaltrohr bezeichnet.

Weil die Feststoffpartikel in der breiartigen Masse vereinzelt und gleichmäßig verteilt vorliegen und keine Ablagerungen von Feststoffpartikeln auf dem Boden der formgebenden Mittel aufgrund des untergemischten Geliermittels auftreten, können neben den Flüssigbestandteilen auch alle Feststoffpartikel der breiartigen Masse mit beschleunigten Elektronen beaufschlagt und somit an den Feststoffpartikeln anhaftende Mikroorganismen inaktiviert werden. Noch weiter erhöht wird dabei die Ausbeute bezüglich des Inaktivierens, wenn die breiartige Masse im Spaltrohr von beiden Seiten durch mindestens zwei gegenüberliegend angeordnete Elektronenstrahlerzeuger mit beschleunigten Elektronen beaufschlagt wird.

Die geringe Dicke eines Spaltrohrs von 1 mm bis 3 mm bringt den Vorteil mit sich, dass zum vollständigen Durchdringen der breiartigen Masse im Spaltrohr nur Elektronenstrahlerzeuger mit geringer Beschleunigungsspannung und Strahlungsleistung und somit relativ preisgünstige Elektronenstrahlerzeuger benötigt werden, was die Wirtschaftlichkeit erfindungsgemäßer Verfahren und Vorrichtungen erhöht. Hierfür sind beispielsweise Elektronenbeschleuniger mit einer Elektronenenergie im Bereich von 130 keV bis 800 keV geeignet.

Bezüglich der Breite eines Spaltrohrs bestehen hingegen keinerlei Einschränkungen. Es muss lediglich gewährleistet sein, dass die gesamte Breite des schichtförmigen Volumens mit beschleunigten Elektronen aus einem oder mehreren Elektronenstrahlerzeugern beaufschlagt wird.

Aufgrund der schmalen Abmaße des Spaltrohrs hinsichtlich der Spaltdicke von 1 mm bis 3 mm sind erfindungsgemäße Verfahren und Vorrichtungen jedoch nicht nur auf das Inaktivieren von Feststoffpartikel enthaltende Massen mit Feststoffpartikeldurchmesser von maximal 1 mm beschränkt. Sollte eine Feststoffpartikel enthaltende Masse Feststoffpartikel mit einem größeren Durchmesser als 1 mm aufweisen, ist vor dem Vermischen mit dem Geliermittel lediglich ein zusätzlicher Verfahrensschritt, nämlich das Zerkleinern der Feststoffpartikel auf Partikel mit einem Durchmesser kleiner als 1 mm, erforderlich. Bei größeren Partikeldurchmessern kann das Zerkleinern gegebenenfalls auch mehrstufig erfolgen. So können auch Feststoffpartikel mit beliebig großem Durchmesser mittels erfindungsgemäßer Verfahren und Vorrichtungen inaktiviert werden. Damit Feststoffpartikel nicht den Spaltkanal verstopfen, ist es hinreichend, wenn die Feststoffpartikel einen Durchmesser von 0,2 mm bis 0,6 mm aufweisen. Die Partikel können jedoch auch auf noch kleinere Abmaße reduziert werden.

Bei einer Ausführungsform wird das schichtförmige Volumen von beiden Seiten mit beschleunigten Elektronen beaufschlagt, wobei die Leistungsparameter der gegenüberliegend angeordneten Elektronenstrahlerzeuger derart eingestellt sind, dass die auf die Feststoffpartikel enthaltende Masse applizierte Elektronenenergie in der Schichtdickenmitte des Volumens ein Maximum aufweist. Strömende Volumen weisen üblicherweise in der Mitte des Volumens eine höhere Strömungsgeschwindigkeit auf als an den Rändern. Werden die Leistungsparameter der gegenüberliegend angeordneten Elektronenstrahlerzeuger derart eingestellt, dass die auf die Feststoffpartikel enthaltende Masse applizierte Elektronenenergie in der Schichtdickenmitte des Volumens ein Maximum aufweist, so wird die über die Spaltdicke applizierte Energiedosis homogenisiert, d. h. die über die Spaltdicke verteilten Bestandteile der Feststoffpartikel enthaltenden Masse werden dann mit einer gleichmäßigeren Energiedosis beaufschlagt.

Bei einer weiteren Ausführungsform wird beim Vermischen der Feststoffpartikel enthaltenden Masse und dem Geliermittel Luft, Sauerstoff, eine Sauerstoff abgebende Substanz, wie beispielsweise ein Peroxid (z. B. H₂O₂), oder ein anderes reaktives Gas beigemischt und dadurch ein pumpfähiger Schaum erzeugt, welcher dann durch die formgebenden Mittel transportiert und im Spaltrohr mit beschleunigten Elektronen beaufschlagt wird. Die Beimischung eines Reaktivgases oder einer Sauerstoff abgebenden Substanz erhöht die Strahlensensibilität von Mikroorganismen, wodurch die zum Inaktivieren erforderliche zu übertragende Energiedosis verringert werden kann.

Eine Zufuhr von Wärmeenergie vor dem Beaufschlagen mit beschleunigten Elektronen führt ebenfalls dazu, dass die Strahlensensibilität von Mikroorganismen erhöht wird. Dabei kann die Wärme entweder durch Reibungseffekte beim Misch- und Homogenisierungsprozess entstehen oder durch zusätzliche Wärme abgebende physikalische Energiequellen, wie beispielsweise Wärmestrahler, erzeugt werden. Alternativ kann die Wärme aber auch durch die Beimischung von Zusatzstoffen erzeugt werden, die eine exotherme chemische Reaktion mit Bestandteilen der Feststoffpartikel enthaltenden Masse und/oder dem Geliermittel bewirken.

Als Geliermittel sind beispielsweise Methylzellulose, Xanthan, Johannisbrotkernmehl oder Natriumalginat geeignet. Es kann jedoch auch jedes andere Mittel, welches eine gelierende Wirkung hervorruft, verwendet werden.

Nach dem Beaufschlagen mit beschleunigten Elektronen liegt eine inaktivierte und für den Menschen nicht mehr gesundheitsschädigende Masse vor, die nicht mehr einer kostenintensiven Entsorgung als Sondermüll unterzogen werden muss. Die Masse aus flüssigen und festen Bestandteilen kann im nun inaktivierten Zustand wie nicht kontaminierter Abfall (wie beispielsweise Hausmüll) entsorgt oder aber auch einer Wiederverwertung zugeführt werden. So kann der Masse beispielsweise durch einen Granulationsprozess das Wasser entzogen werden. Das auf diese Weise entstehende Granulat kann dann in einem Wiederverwertungsprozess integriert oder aber in der vorliegenden Form als Granulat auch problemlos über längere Zeit gelagert werden.

Im Spaltrohr selbst kommt ein weiterer Vorteil des untergemischten Geliermittels zum Tragen. Das Geliermittel bildet nämlich eine Art Schmierfilm um die Partikel der Feststoffpartikel enthaltenden Masse aus, was die Abrasion an den Wänden des Spaltrohres zumindest wesentlich vermindert oder sogar vollständig ausschließt. Dadurch ist es möglich, den Bereich eines Spaltrohres, durch den von einem Elektronenbeschleuniger ausgesandte Elektronen das Spaltrohr durchdringen und auf die mit Elektronen zu beaufschlagende Masse treffen, als dünne Metallmembran in Form eines Elektroneneintrittsfensters (ähnlich dem Elektronenaustrittsfenster eines Elektronenstrahlerzeugers) auszubilden und dennoch eine hohe Lebensdauer der Metallmembran zu gewährleisten.

So kann ein Elektroneneintrittsfenster beispielsweise als Metallmembran mit einer Dicke von 8 µm bis 35 µm ausgebildet sein. Als Elektroneneintrittsfenster sind dünne Folien aus Titan besonders geeignet. Aufgrund der geringen Dicke einer solchen Metallmembran wird eine hohe Transparenz für die Elektronen erzielt, was den Einsatz von Elektronenstrahlerzeugern mit einer relativ geringen Beschleunigungsspannung in einem Bereich von 150 kV bis 800 kV ermöglicht.

Bei einer weiteren Ausführungsform weist eine solche als Elektroneneintrittsfenster im Spaltrohr ausgebildete Metallmembran eine Stützkonstruktion auf, um den innerhalb des Spaltrohres wirkenden Druck, der beim Pumpen der Masse durch das Spaltrohr entsteht, aufzunehmen. Eine solche Stützkonstruktion kann beispielsweise als gebohrte Platte, Lamellen, Wabenstruktur, gewölbte Drähte oder Stäbe ausgebildet sein. Beim Verwenden einer Stützkonstruktion sollte jedoch darauf geachtet werden, dass keine Todbereiche für den Elektronenstrahl entstehen, sondern die Masse im Spaltrohr dennoch über die gesamte Breite des Spaltrohres gleichmäßig mit Elektronen beaufschlagt wird.

Die hohe Energie der beschleunigten Elektronen wird vom Spaltrohr und insbesondere von einem darin integrierten Elektroneneintrittsfenster teilweise in Form von Wärmeenergie aufgenommen. Daher ist es vorteilhaft, wenn das Spaltrohr über geeignete Wärme abführende Mittel verfügt. So können beispielsweise im Spaltrohr oder/und in der Stützkonstruktion für ein Elektroneneintrittsfenster Kühlkanäle integriert sein, durch die ein Kühlmittel fließt.

Bei einer bevorzugten Ausführungsform sind Spaltrohr und Elektronenstrahlerzeuger derart ausgebildet und zueinander ausgerichtet, dass das Elektronenaustrittsfenster des Elektronenstrahlerzeugers und das zugehörige Elektroneneintrittsfenster im Spaltrohr mit einem Maß voneinander beabstandet sind. Im räumlichen Bereich zwischen Elektronenaustrittsfenster und Elektroneneintrittsfenster kann beispielsweise ein Unterdruck in einem Bereich von 200 mbar bis 900 mbar erzeugt und mittels einer Messeinrichtung überwacht werden. Auf diese Weise können Beschädigungen am Elektronenaustrittsfenster und am Elektroneneintrittsfenster erfasst und eine dann erforderliche Stilllegung der Vorrichtung ausgelöst werden. Steigt nämlich der Druck im Bereich zwischen Elektronenaustrittsfenster und Elektroneneintrittsfenster an, ist dies ein Indiz für ein defektes Elektroneneintrittsfenster im Spaltkanal, wodurch sich dann der Überdruck im Inneren des Spaltrohres auch auf den Bereich zwischen Elektronenaustrittsfenster und Elektroneneintrittsfenster erstreckt. Ein Absinken des Drucks im Bereich zwischen Elektronenaustrittsfenster und Elektroneneintrittsfenster weist hingegen auf ein defektes Elektronenaustrittsfenster des Elektronenstrahlerzeugers hin, weil dann das Vakuum des Elektronenstrahlerzeugers auch den Bereich zwischen Elektronenaustrittsfenster und Elektroneneintrittsfenster mit evakuiert.

Alternativ kann im Bereich zwischen Elektronenaustrittsfenster und Elektroneneintrittsfenster aber auch ein Überdruck aufrechterhalten und mit Messmitteln überwacht werden.

### Ausführungsbeispiel

Die Erfindung wird nachfolgend anhand eines bevorzugten Ausführungsbeispiels näher erläutert. Die Fig. zeigen:
- Fig. 1: eine schematische Darstellung einer erfindungsgemäßen Vorrichtung zum Inaktivieren von Abfällen aus der Pharmaindustrie;
- Fig. 2: das Spaltrohr 15 aus Fig. 1 mit oberem Elektroneneintrittsfenster in einer perspektivischen Explosionsdarstellung;
- Fig. 3: eine perspektivische Darstellung des Spaltrohres 15 aus Fig. 1 im zusammengesetzten Zustand;
- Fig. 4: eine schematische Querschnittsdarstellung des Spaltrohres 15 aus Fig. 1 mit oberem und unterem Elektronenstrahlerzeuger;
- Fig. 5a+b: die relative Dosisverteilung der von Elektronenstrahlerzeugern 17 und 18 beschleunigten Elektronen.

Beim Herstellen von Seren für Grippeschutzimpfungen werden Hühnereier mit Grippeviren infiziert. Nach einiger Zeit des Bebrütens und nachdem sich die Viren in den Eiern um ein Vielfaches vermehrt haben, wird nun eine Flüssigkeit "geerntet", d. h. aus den Eiern extrahiert, und in weiteren Verarbeitungsschritten werden daraus Seren zur Schutzimpfung gewonnen. Zurück bleiben Eierrückstände, bestehend aus Eierschalen, Embryonen und ggf. noch Eiweiß und Eigelb, welche jeweils mit Grippeviren kontaminiert sind.

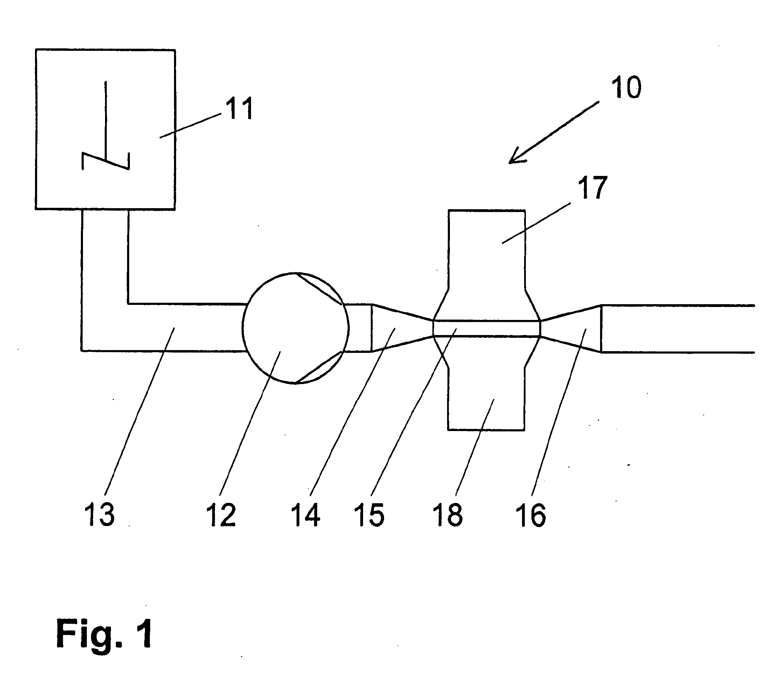

Mit einer in Fig. 1 schematisch dargestellten erfindungsgemäßen Vorrichtung 10 sollen diese Eierrückstände inaktiviert werden, d. h. es soll zumindest dafür gesorgt werden, dass die an den Eierrückständen anhaftenden Grippeviren nicht mehr vermehrungsfähig sind, wodurch dann keine gesundheitsschädigende Gefährdung mehr für den Menschen von den Eierrückständen ausgeht.

In einem ersten Schritt werden die Feststoffpartikel der aus festen und flüssigen Eierrückständen bestehenden Masse mit graphisch nicht dargestellten Mitteln in mehreren Stufen auf eine letztendliche Partikelgröße von maximal 0,5 mm zerkleinert und die festen und flüssigen Eierrückstände anschließend in einem Behältnis 11 mit als Geliermittel fungierender Methylzellulose vermischt.

Das Vermischen erfolgt dabei derart, dass einerseits die Feststoffpartikel in der entstehenden breiartigen und mit Viren kontaminierten Masse dispergiert vorliegen und andererseits die breiartige Masse eine Konsistenz aufweist, in der die Feststoffpartikel "in Schwebe" gehalten werden, also kein Sedimentieren der Feststoffpartikel erfolgt. Beim Vermischen wird die kontaminierte Masse gleichzeitig mit 10 Vol. % Luft aufgeschäumt und auf eine Temperatur von 50 °C erwärmt.

Mittels einer Pumpeinrichtung 12 wird die nun homogenisierte breiartige Masse zunächst durch Rohrleitungen 13 und anschließend durch formgebende Mittel transportiert. Die formgebenden Mittel umfassen ein Übergangsstück 14, in welchem ein kreisförmiger Rohrquerschnitt auf einen rechteckigen Querschnitt transformiert wird; ein Spaltrohr 15, in welchem der rechteckige Querschnitt beibehalten wird und die kontaminierte Masse somit ein gleichbleibendes schichtförmiges Volumen mit 2 mm Schichtdicke und 100 mm Breite annimmt, und ein weiteres Übergangsstück 16, in welchem der rechteckige Rohrquerschnitt wieder auf einen runden Querschnitt zurück transformiert wird. Das erneute Verändern des Querschnittes nach dem Durchqueren des Spaltrohres 15 ist nicht erfindungswesentlich, vereinfacht jedoch beim konkreten Ausführungsbeispiel die sich nach Spaltrohr 15 anschließende Rohrführung.

Im Bereich des Spaltrohres 15 wird die nun schichtförmige Masse von beiden Seiten mittels gegenüberliegend angeordneter Elektronenstrahlerzeuger 17 und 18 mit beschleunigten Elektronen beaufschlagt und dadurch inaktiviert, d. h. die in der breiartigen Masse vorhandenen bzw. an den Feststoffpartikeln anhaftenden Grippeviren werden abgetötet bzw. sind nach dem Beaufschlagen mit beschleunigten Elektronen zumindest nicht mehr vermehrungsfähig und stellen somit keine gesundheitsschädigende Gefahr mehr für den Menschen dar.

Die beiden Elektronenstrahlerzeuger 17 und 18 umfassen jeweils einen sogenannten Axialstrahler und eine zugehörige Strahlablenkung mit Ablenksteuerung, mittels der der vom Axialstrahler erzeugte Elektronenstrahl abgelenkt wird. Daher wirken beide Elektronenstrahlerzeuger 17, 18 als Flächenstrahlerzeuger.

Nach dem Durchqueren der formgebenden Mittel 14, 15, 16 und dem Beaufschlagen mit beschleunigten Elektronen wird der nun inaktivierten Masse in einem Granulationsprozess mittels nicht dargestellter Einrichtungen das Wasser entzogen. Als Ergebnis entsteht hierbei ein hochwertiges Granulat, welches vorwiegend aus Eiweiß besteht, und einer Wiederverwertung, wie beispielsweise als Futtermittel oder als Beimischung bei der Düngemittelherstellung, zugeführt werden kann.

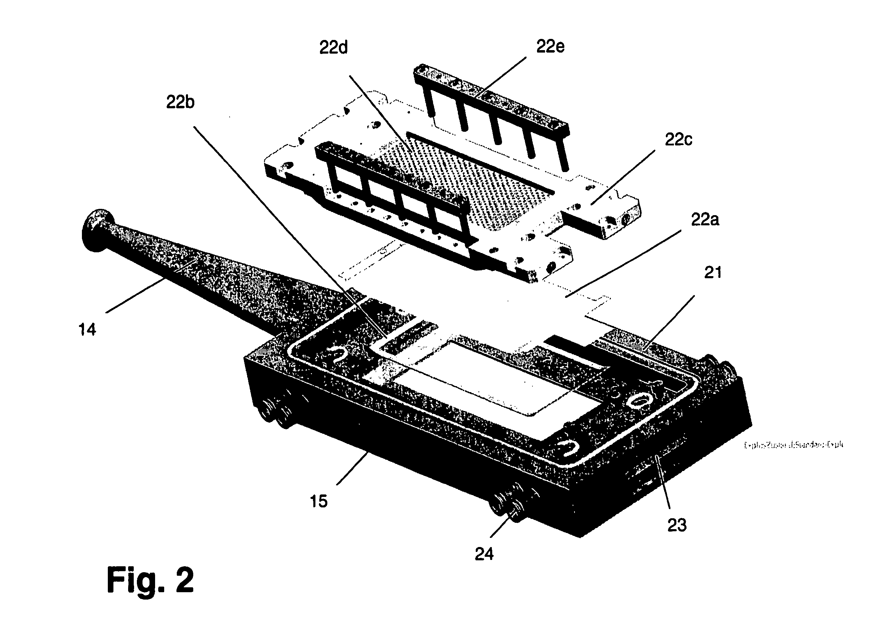

In Fig. 2 ist das Spaltrohr 15 aus Fig. 1 mit eingangsseitigem Übergangsstück 14 schematisch in einer Perspektive und teilweise als Explosionsdarstellung abgebildet. Das Spaltrohr 15 umfass einen Grundkörper 21 sowie ein oberes Elektroneneintrittsfenster 22 und ein in Fig. 2 nicht dargestelltes aber ebenso wie Elektroneneintrittsfenster 22 ausgebildetes unteres Elektroneneintrittsfenster. Ebenso zu erkennen ist in Fig. 2 die Austrittsseite des Spaltes 23, der das Spaltrohr 15 durchzieht und durch den die kontaminierte Masse gepumpt wird.

Das Elektroneneintrittsfenster 22 umfasst eine 25 µm dicke Titanfolie 22a, welche im zusammengebauten Zustand einen Teil der Innenwandung des Spaltrohres bildet und somit im direkten Kontakt mit der durch das Spaltrohr gepumpten Masse steht; ein zwischen Grundkörper 21 und Titanfolie 22a angeordnetes Dichtelement 22b; eine Stützkonstruktion 22c, welche im Bereich 22d, über den der von Elektronenstrahlerzeuger 17 erzeugte Elektronenstrahl abgelenkt wird, eine Lochstruktur aufweist; sowie Befestigungselemente 22e, mittels denen die Stützkonstruktion 22c, die Titanfolie 22a und das Dichtelement 22b an den Grundkörper 21 gepresst und befestigt werden. Mit der am Grundkörper 21 befestigten Stützkonstruktion 22c wird der Druck aus dem Inneren des Spaltrohres aufgefangen, der entsteht, wenn die kontaminierte Masse durch das Spaltrohr 15 gepumpt wird. Die dünne Titanfolie 22a allein könnte diesem Druck nicht standhalten bzw. würde sich aufgrund des Druckes zumindest nach außen wölben und somit die Dicke des Spaltes 23 erhöhen, was jedoch nicht erwünscht ist, weil dies zu einem inhomogeneren Bestrahlungsergebnis an der kontaminierten Masse, über die Schichtdicke des Volumens betrachtet, führen würde.

Ebenfalls ersichtlich sind in Fig. 2 mehrere Anschlüsse 24 für Kühlmittelleitungen. Der Grundkörper 21 ist von mehreren nicht dargestellten Kanälen durchzogen, durch welche das Kühlmittel aus den Kühlmittelleitungen geführt wird, womit die Wärmeenergie, die beim Durchtritt der beschleunigten Elektronen durch die beiden Elektroneneintrittsfenster entsteht und sich aufgrund von Wärmeleitung im gesamten Spaltrohr ausbreitet, abgeführt werden kann.

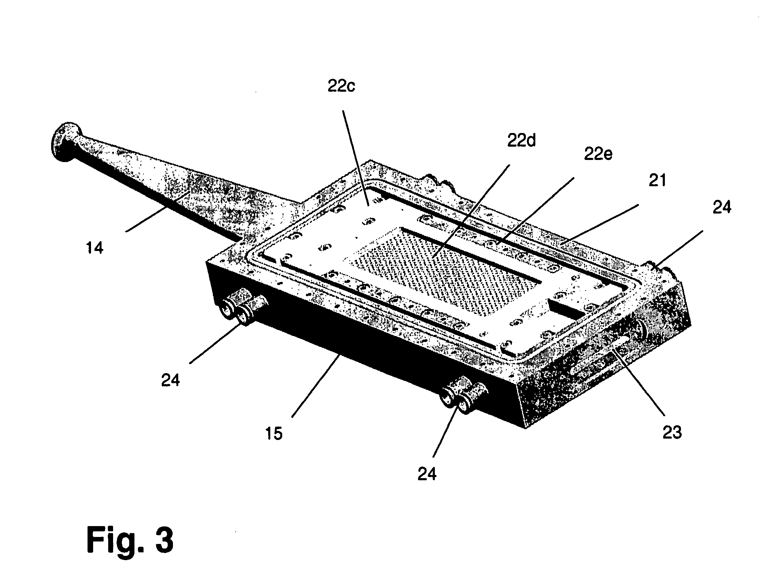

Fig. 3 zeigt noch einmal schematisch das Spaltrohr 15 mit eingangsseitigem Übergansstück 14 in einer perspektivischen Darstellung, jedoch zeigt Fig. 3 das Spaltrohr 15 im zusammengesetzten Zustand, so dass in Fig. 3 nicht mehr alle zu Fig. 2 beschriebenen Bauelemente des Spaltrohres 15 erkenntlich sind.

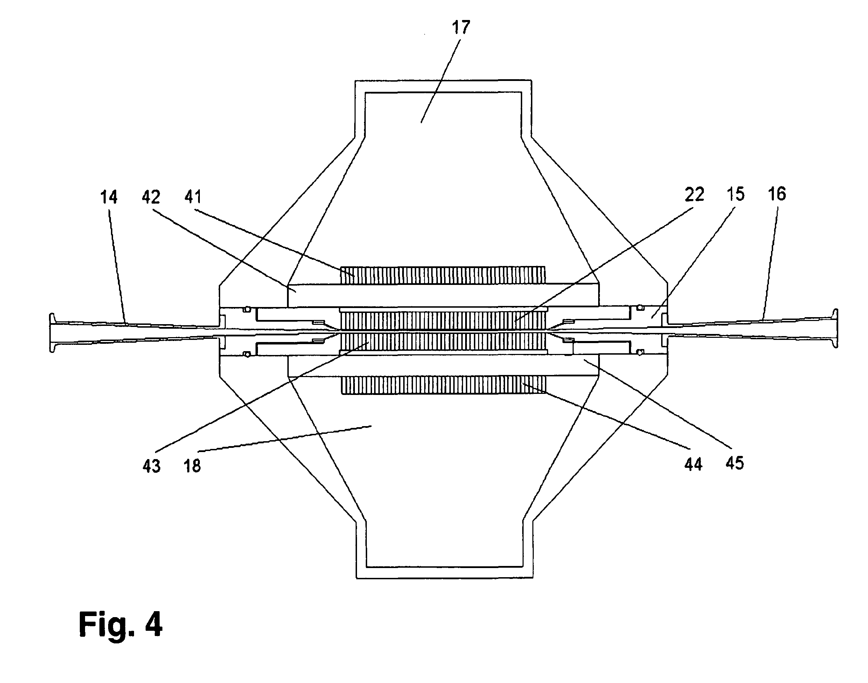

Eine schematische Querschnittsdarstellung des Spaltrohres 15 mit Übergangsstücken 14 und 16 sowie angeflanschten Elektronenstrahlerzeugern 17 und 18 ist in Fig. 4 abgebildet. Auf dem Spaltrohr 15 ist der Elektronenstrahlerzeuger 17 befestigt, der ein Elektronenaustrittsfenster 41 umfasst. Der Elektronenstrahlerzeuger 17 ist dabei derart positioniert, dass sein ebenes Elektronenaustrittsfenster 41 parallel gegenüberliegend zum ebenfalls ebenen Elektroneneintrittsfenster 22 angeordnet ist. Der Vollständigkeit halber sein noch einmal erwähnt, dass das Elektronenfenster 22 die Teilelemente 22a, 22b, 22c, 22d und 22e umfasst. Beide Fenster 41 und 22 sind jedoch mit einem Maß von 30 mm voneinander beabstandet und begrenzen daher einen Bereich 42, der mit Stickstoff auf einen Absolutwert von 800 mbar gefüllt ist. Dieser Unterdruck wird mit nicht dargestellten Mitteln aufrechterhalten.

Die vom Elektronenstrahlerzeuger 17 erzeugten und beschleunigten Elektronen treten daher zunächst durch das Elektronenaustrittsfenster 41, überbrücken den Bereich 42, treten anschließend durch das Elektroneneintrittsfenster 22 und beaufschlagen die kontaminierte Masse, die durch das Spaltrohr 15 gepumpt wird.

Die Druckverhältnisse im Bereich 42 werden mit einer ebenfalls nicht dargestellten Messeinrichtung überwacht. Beim Überschreiten eines ersten Druckschwellwertes wird ebenso wie beim Unterschreiten eines kleineren zweiten Druckschwellwertes ein Warnsignal ausgelöst und/oder die gesamte Anlage abgeschaltet, denn das Überschreiten des ersten Druckschwellwertes ist ein Hinweis auf ein undichtes bzw. defektes Elektroneneintrittsfenster 22 und ein Unterschreiten des zweiten Schwellwertes ist ein Hinweis auf ein undichtes bzw. defektes Elektronenaustrittsfenster 41.

Spiegelsymmetrisch zu Elektronenstrahlerzeuger 17 und mit gleichem Aufbau und gleicher Wirkungsweise ist Elektronenstrahlerzeuger 18 am unteren Elektroneneintrittsfenster 43 des Spaltrohres 15 angeflanscht. Auch hier sind Elektronenaustrittsfenster 44 des Elektronenstrahlerzeugers 18 und zugehöriges Elektroneneintrittsfenster 43 des Spaltrohres 15 mit einem Maß beabstandet und begrenzen somit einen Bereich 45, der mit Stickstoff gefüllt ist und dessen Unterdruckverhältnisse mittels einer Messeinrichtung überwacht werden.

Das Führen der kontaminierten Masse durch das Spaltrohr 15 erfolgt kontinuierlich zwischen den beiden Elektroneneintrittsfenstern 22, 43 hindurch, wobei das gesamte Volumen der hindurchströmenden Masse mit Elektronenenergie beaufschlagt wird. In der Mitte des Strömungsquerschnitts des Kanals 23 herrscht naturgemäß die höchste Strömungsgeschwindigkeit im Vergleich zur Grenzfläche an der Kanalwand (Elektroneneintrittsfenster), wodurch bei homogenem Energieeintrag in der Spaltmitte die geringste Energiedosis übertragen würde. Durch die Wahl einer optimalen Beschleunigungsspannung für beide Elektronenbeschleuniger 17, 18 wird dieses Minimum kompensiert.

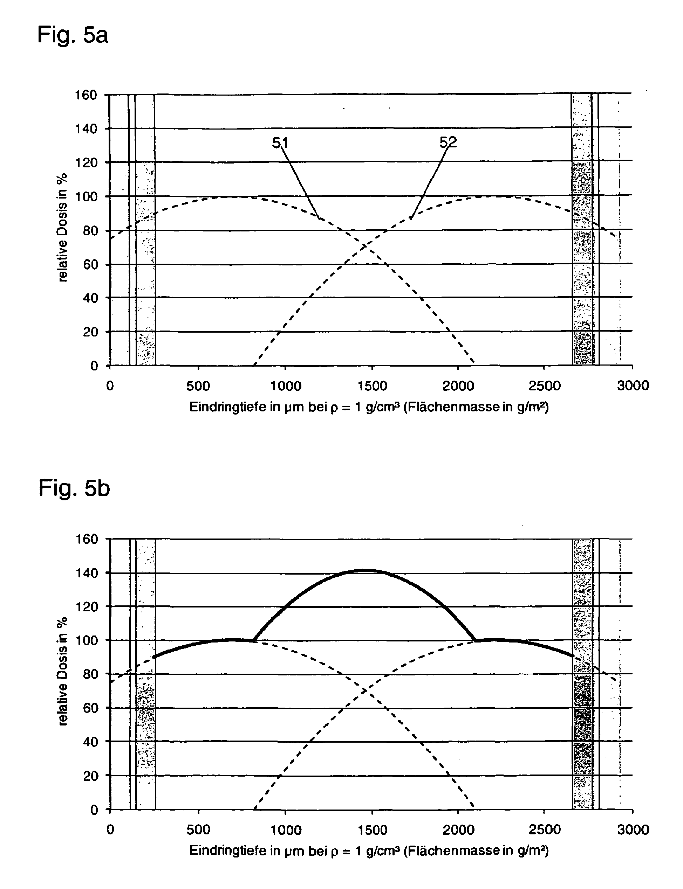

Fig. 5a zeigt graphisch die beispielhafte relative Tiefendosisverteilung einer erfindungsgemäßen Anordnung zweier Elektronenbeschleuniger 17, 18 gemäß Fig. 4 mit einer Dicke der zu den Elektronenaustrittsfenstern 41, 44 gehörenden Titanfolien von 25 µm bei einer Beschleunigungsspannung von 500 kV und einem Maß zwischen den Elektronenaustrittsfenstern 41, 44 und dem jeweils zugehörigen Elektroneneintrittsfenster 22, 43 von jeweils 30 mm, einer Dicke der Titanfolien der Elektroneneintrittsfenster 22, 43 von jeweils 25 µm und einer Schichtdicke der kontaminierten Masse von 2 mm im Kanal 23 des Spaltrohrs 15.

Die gestrichelte Kurve 51 stellt die Verteilung der durch Elektronenbeschleuniger 17 erzeugten Energiedosis über der Eindringtiefe der von ihm beschleunigten Elektronen dar.

Der Nullpunkt der horizontalen Achse ist gleichbedeutend mit jener Position, an der die beschleunigten Elektronen des Elektronenstrahlerzeugers 17 auf die Titanfolie seines Elektronenaustrittsfensters 41 auftreffen. Der erste dunkle Balken links stellt daher die Titanfolie des Elektronenaustrittsfensters 41 und der zweite dunkle Balken die Titanfolie von Elektroneneintrittsfenster 22 dar.

Bei Austritt aus der Titanfolie von Elektronenaustrittsfenster 41 wird eine Energiedosis von etwa 80 % erzielt. Mit zunehmender Eindringtiefe steigt diese Dosis weiter an, bis sie bei etwa 700 µm (hier befinden sich die Elektronen schon innerhalb der zu beaufschlagenden Masse im Spaltrohr 15) ihr Maximum erzielt. Mit weiter zunehmender Eindringtiefe nimmt jetzt die Energiedosis ab, bis die Energie der Elektronen bei einer Eindringtiefe, die etwa 2100 µm bei Dichte 1 g/cm³ entspricht, auf den Wert Null abgefallen ist. Die beschleunigten Elektronen des Elektronenstrahlerzeugers 17 erreichen also nicht das gegenüberliegende Elektroneneintrittsfenster 43 sondern sie geben ihre Energie schon vollständig ab, noch bevor die kontaminierte Masse in deren Schichtdicke vollständig durchdrungen ist. Von der anderen Seite wird jedoch die kontaminierte Masse mit den beschleunigten Elektronen vom Elektronenstrahlerzeuger 18 beaufschlagt.

Die beiden dunklen Balken auf der rechten Seite sind spiegelsymmetrisch den beiden Titanfolien von Elektronenaustrittsfenster 44 und Elektroneneintrittsfenster 43 zuzuordnen. Die Verteilung der durch Elektronenbeschleuniger 18 erzeugten Energiedosis über der Eindringtiefe der von ihm beschleunigten Elektronen ist mit der gestrichelten Kurve 52 dargestellt.

Es ist zu erkennen, dass ein Mittenbereich bezüglich der Schichtdicke der zu bestrahlenden Masse von beiden Seiten mit beschleunigten Elektronen beaufschlagt wird. Daher addiert sich in diesem Bereich auch die Energiedosis. Die Kurve aus der Summe der von beiden Seiten eingetragenen Energiedosis ist als durchgehende Kurve in Fig. 5b dargestellt. Diese weist in der Mitte des Spaltrohres ein Maximum auf. Durch diese Dosisüberhöhung wird die erhöhte Strömungsgeschwindigkeit in der Spaltrohrmitte kompensiert und es resultiert eine weitgehend homogene Energiedosis über das gesamte Volumen der durch das Spaltrohr strömenden Masse.

Das Ausführungsbeispiel ist zwar konkret in Bezug auf das Inaktivieren von Bruteiabfällen aus der Pharmaindustrie beschrieben, erfindungsgemäße Verfahren und Vorrichtungen sind jedoch auch zum Inaktivieren aller mikrobiologisch kontaminierten Massen geeignet, deren Feststoffbestandteile auf eine maximale Partikelgröße von 1 mm zerteilbar sind.

## Patentansprüche

1. Verfahren zum Inaktivieren einer mikrobiologisch kontaminierten und Feststoffpartikel enthaltenden Masse mittels beschleunigter Elektronen, **gekennzeichnet durch** folgende Verfahrensschritte:
a) Vermischen der Feststoffpartikel enthaltenden Masse mit einem Geliermittel;
b) Transportieren der mit Geliermittel, vermischten Feststoffpartikel enthaltenden Masse **durch** formgebende Mittel (14; 15; 16), in denen die Masse zumindest in einem Bereich (15) zu einem schichtförmigen Volumen mit einer Schichtdicke von 1 bis 3 mm geformt wird;
c) Beaufschlagen der mit Geliermittel vermischten Feststoffpartikel enthaltenden Masse im schichtförmigen volumenbereich mit beschleunigten Elektronen.

2. Verfahren nach Anspruch 1, **dadurch gekennzeichnet, dass** die Feststoffpartikel vor dem Zusatz des Geliermittels auf eine Partikelgröße von maximal 1 mm und vorzugsweise auf eine Partikelgröße in einem Bereich von 0,2 mm bis 0,6 mm zerkleinert werden.

3. Verfahren nach einem der Ansprüche 1 oder 2, **dadurch gekennzeichnet, dass** die Feststoffpartikel enthaltende Masse mit einer Flüssigkeit vermischt wird.

4. Verfahren nach einem der vorhergehenden Ansprüche, **dadurch gekennzeichnet, dass** die Masse durch die formgebenden Mittel (14; 15; 16) gepumpt, gesaugt oder gepresst wird.

5. Verfahren nach einem der vorhergehenden Ansprüche, **dadurch gekennzeichnet dass** die Masse im schichtförmigen Volumenbereich von mindestens einer Seite mittels mindestens eines Eiektronenstrahl,erzeugers mit beschleunigten Elektronen beaufschlagt wird.

6. Vorfahren nach Anspruch 5, **dadurch gekennzeichnet, dass** die Masse im schichtförmigen Volumenbereich von zwei Seiten mittels mindestens zwei gegenüberliegend angeordneten Elektronenstrahlerzeugern (17; 18) mit beschleunigten Elektronen beaufschlagt wird.

7. Verfahren nach einem der vorhergehenden Ansprüche, **dadurch gekennzeichnet, dass** der Feststoffpartikel enthaltenden Masse nach dem Beaufschlagen mit beschleunigten Elektronen Flüssigkeit entzogen wird.

8. Vorrichtung zum Inaktivieren einer mikrobiologisch kontaminierten und Feststoffpartikel enthaltenden Masse mittels beschleunigter Elektronen, umfassend Fördermittel (12) zum Transportieren der Feststoffpartikel enthaltenden Masse durch formgebende Mittel (14; 15; 16), in denen die Masse Zumindest in einem Bereich (15) zu einem schichtförmigen Volumen mit einer Schichtdicke von 1-3 mm formbar ist, und
mindestens einen Elektronenbeschleuniger (17; 18), mittels dem die Feststoffpartikel enthaltende Masse in einem schichtförmigen Volumenbereich (15) mit beschleunigten Elektronen beaufschlagbar ist, **gekennzeichnet durch**
erste Mittel (11) zum Vermischen der Feststoffpartikel enthaltenden Masse mit einem Geliermittel und zum Dispergieren der Feststoffpartikel;
formgebende Mittel (14; 15; 16), in denen die mit Geliermittel vermischte Masse zumindest im Bereich (15) zu einem schichtförmigen Volumen mit einer Schichtdicke von 1 bis 3 mm formbar ist;
wobei ein Bereich der formgebenden Mittel als Elektroneneintrittsfenster (22) ausgebindet ist, **durch** welches Elektronen vom Elektronenbeschleuniger (17) zur Feststoffpartikel enthaltenden Masse gelangen und
wobei das Elektroneneintrittsfenster (22) vom Elektronenaustrfttsfenster (41) des zugehörigen Elektronenbeschleunigers (17) mit einem Maß beabstandet ist.

9. Vorrichtung nach Anspruch 8, **dadurch gekennzeichnet, dass** mindestens zwei Elektronenbeschleüniger (17; 18) gegenüberliegend angeordnet sind, so dass die Feststoffpartikel enthaltende Masse von zwei Seiten mit beschleunigten Elektronen beaufschlagbar ist.

10. Vorrichtung nach einem der Ansprüche 8 bis 9, **dadurch gekennzeichnet, dass** das Elektroneneintrittsfenster (22) eine Metallmembran (22a) mit einer Dicke von 8 µm bis 35 µm und eine Stützeinrichtung (22c) umfasst:

11. Vorrichtung nach einem der Ansprüche 8 bis 10, **dadurch gekennzeichnet, dass** die Elektronemenergie des Elektronenbeschleunigers (17) im Bereich von 130 keV bis 800 keV liegt.

12. Vorrichtung nach einem der Ansprüche 8 bis 111, **dadurch gekennzeichnet, dass** der Bereich (42) zwischen Elektronenaustrittsfenster (41) und Etektroneneintrittsfenster (22) einen Unterdruck oder einen Überdruck aufweiset.

13. Vorrichtung nach einem der Ansprüche 8 bis 12, **gekennzeichnet durch** eine Messeinrichtung, mittels der eine Druckveränderung im Bereich zwischen Elektronenaustrittsfenster (41) und Elektroneneintrittsfenster (22) erfassbar ist.

## Claims

1. Method for inactivating a microbiologically contaminated, solid-particle-containing material using accelerated electrons, **characterized by** the following method steps:
a) mixing the solid-particle-containing material with a gelling agent;
b) transporting the solid-particle-containing material that is mixed with the gelling agent through shaping means (14; 15; 16), in which the material is shaped at least in a region (15) into a layer-shaped volume having a layer thickness of 1 to 3 mm;
c) subjecting the solid-particle-containing material that is mixed with the gelling agent in the layer-shaped volume region to accelerated electrons.

2. Method according to Claim 1, **characterized in that** the solid particles are reduced in size to a particle size of at most 1 mm and preferably to a particle size ranging from 0.2 mm to 0.6 mm before the gelling agent is added.

3. Method according to one of Claims 1 or 2, **characterized in that** the solid-particle-containing material is mixed with a liquid.

4. Method according to one of the preceding claims, **characterized in that** the material is pumped, sucked or pressed through the shaping means (14; 15; 16).

5. Method according to one of the preceding claims, **characterized in that** the material is subjected to accelerated electrons in the layer-shaped volume region from at least one side by at least one electron-beam generator.

6. Method according to Claim 5, **characterized in that** the material is subjected to accelerated electrons in the layer-shaped volume region from two sides by at least two electron beam generators (17; 18) which are arranged opposite each other.

7. Method according to one of the preceding claims, **characterized in that** liquid is removed from the solid-particle-containing material after it has been subjected to accelerated electrons.

8. Apparatus for inactivating a microbiologically contaminated, solid-particle-containing material using accelerated electrons, comprising
conveying means (12) for transporting the solid-particle-containing material through shaping means (14; 15; 16), in which the material is shaped at least in a region (15) into a layer-shaped volume having a layer thickness of 1-3 mm and
at least one electron accelerator (17; 18), with which the solid-particle-containing material is subjectable to accelerated electrons in a layer-shaped volume region (15), **characterized by**
first means (11) for mixing the solid-particle-containing material with a gelling agent and for dispersing the solid particles;
shaping means (14; 15; 16) in which the material which has been mixed with gelling agent is shapeable at least in region (15) into a layer-shaped volume having a layer thickness of 1 to 3 mm;
wherein a region of the shaping means is configured as an electron entry window (22) through which electrons pass from the electron accelerator (17) to the solid-particle-containing material and
wherein the electron entry window (22) is spaced apart by a distance from the electron exit window (41) of the associated electron accelerator (17).

9. Apparatus according to Claim 8, **characterized in that** at least two electron accelerators (17; 18) are arranged opposite each other such that the solid-particle-containing material is subjectable by accelerated electrons from two sides.

10. Apparatus according to one of Claims 8 to 9, **characterized in that** the electron entry window (22) comprises a metal membrane (22a) having a thickness of 8 µm to 35 µm and a support device (22c).

11. Apparatus according to one of Claims 8 to 10, **characterized in that** the electron energy of the electron accelerator (17) ranges from 130 keV to 800 keV.

12. Apparatus according to one of Claims 8 to 11, **characterized in that** the region (42) between the electron exit window (41) and electron entry window (22) has a negative or positive pressure.

13. Apparatus according to one of Claims 8 to 12, **characterized by** a measuring device, by way of which a pressure change in the region between the electron exit window (41) and the electron entry window (22) is detectable.

## Revendications

1. Procédé d'inactivation d'une masse microbiologiquement contaminée et contenant des particules de matière solide à l'aide d'électrons accélérés, **caractérisé par** les étapes consistant à :
a) mélanger la masse contenant des particules de matière solide à un agent de gélification ;
b) transporter la masse contenant des particules de matière solide mélangée à l'agent de gélification à travers des moyens de mise en forme (14 ; 15 ; 16) dans lesquels la masse est mise en forme au moins dans une région (15) en un volume en forme de couche ayant une épaisseur de couche de 1 à 3 mm ;
c) exposer la masse contenant des particules de matière solide mélangée à un agent de gélification dans la région du volume en forme de couche à des électrons accélérés.

2. Procédé selon la revendication 1, **caractérisé en ce que** les particules de matière solide sont finement divisées, avant l'addition de l'agent de gélification, jusqu'à une taille de particule maximale de 1 mm et de préférence, jusqu'à une taille de particule se situant dans la gamme de 0,2 mm à 0,6 mm.

3. Procédé selon l'une quelconque des revendications 1 et 2, **caractérisé en ce que** la masse contenant des particules de matière solide est mélangée à un liquide.

4. Procédé selon l'une quelconque des revendications précédentes, **caractérisé en ce que** la masse est pompée, aspirée ou comprimée à travers les moyens de mise en forme (14 ; 15 ; 16).

5. Procédé selon l'une quelconque des revendications précédentes, **caractérisé en ce que** la masse est exposée à des électrons accélérés dans la région du volume en forme de couche depuis au moins un côté au moyen d'au moins un générateur de faisceau d'électrons.

6. Procédé selon la revendication 5, **caractérisé en ce que** la masse se trouvant dans la région du volume en forme de couche est exposée à des électrons accélérés depuis deux côtés au moyen d'au moins deux générateurs de faisceaux d'électrons (17 ; 18) disposés à l'opposé l'un de l'autre.

7. Procédé selon l'une quelconque des revendications précédentes, **caractérisé en ce que** le liquide est éliminé de la masse contenant des particules de matière solide après l'exposition aux électrons accélérés.

8. Dispositif d'inactivation d'une masse microbiologiquement contaminée contenant des particules de matière solide à l'aide d'électrons accélérés, comprenant des moyens transporteurs (12) destinés à transporter la masse contenant des particules de matière solide à travers des moyens de mise en forme (14 ; 15 ; 16) dans lesquels la masse peut être mise en forme dans une région (15) en un volume sous forme de couche ayant une épaisseur de couche de 1 à 3 mm ; et au moins un accélérateur d'électrons (17 ; 18) au moyen duquel la masse contenant des particules de matière solide peut être introduite dans une région (15) d'un volume en forme de couche à l'aide électrons accélérés, **caractérisé par** :
des premiers moyens (11) destinés à mélanger la masse contenant des particules de matière solide à un agent de gélification et à disperser les particules de matière solide ;
des moyens de mise en forme (14 ; 15 ; 16) dans lesquels la masse mélangée à l'agent de gélification peut être mise en forme dans au moins une région (15) en un volume en forme de couche ayant une épaisseur de couche de 1 à 3 mm ;
dans lequel une région des moyens de mise en forme est réalisée sous la forme d'une fenêtre d'entrée d'électrons (22) à travers laquelle des électrons provenant de l'accélérateur d'électrons (17) peuvent atteindre la masse contenant des particules de matière solide ; et
dans lequel la fenêtre d'entrée d'électrons (22) est espacée d'une fenêtre de sortie d'électrons (41) de l'accélérateur d'électrons (17) associé.

9. Dispositif selon la revendication 8, **caractérisé en ce qu'**au moins deux accélérateurs d'électrons (17; 18) sont disposés l'un en face de l'autre de manière à ce que la masse contenant des particules de matière solide puisse être exposée depuis les deux côtés à des électrons accélérés.

10. Dispositif selon l'une quelconque des revendications 8 à 9, **caractérisé en ce que** la fenêtre d'entrée d'électrons (22) comprend une membrane métallique (22a) ayant une épaisseur de 8 µm à 35 µm et un dispositif de support (22c).

11. Dispositif selon l'une quelconque des revendications 8 à 10, **caractérisé en ce que** l'énergie des électrons de l'accélérateur d'électrons (17) se situe dans la gamme de 130 Kev à 800 Kev.

12. Dispositif selon l'une quelconque des revendications 8 à 11, **caractérisé en ce que** la région (42) située entre la fenêtre de sortie d'électrons (41) et la fenêtre d'entrée d'électrons (22) présente une dépression ou une surpression.

13. Dispositif selon l'une quelconque des revendications 8 à 12, **caractérisé par** un dispositif de mesure au moyen duquel une modification de la pression peut être détectée dans la région située entre la fenêtre de sortie d'électrons (41) et la fenêtre d'entrée d'électrons (22).
